(19) Europäisches Patentamt
European Patent Office
Office européen des brevets

(11) EP 2 135 553 A1

(12) EUROPEAN PATENT APPLICATION
published in accordance with Art. 153(4) EPC

(43) Date of publication:
23.12.2009 Bulletin 2009/52

(51) Int Cl.:
A61B 6/02 (2006.01)   G03B 42/02 (2006.01)

(21) Application number: 08715396.1

(22) Date of filing: 02.04.2008

(86) International application number:
PCT/CN2008/070664

(87) International publication number:
WO 2008/110126 (18.09.2008 Gazette 2008/38)

(84) Designated Contracting States:
AT BE BG CH CY CZ DE DK EE ES FI FR GB GR
HR HU IE IS IT LI LT LU LV MC MT NL NO PL PT
RO SE SI SK TR

(30) Priority: 14.03.2007 CN 200710027152

(71) Applicants:
• Zhang, Yingguang
Guangzhou, Guangdong 510060 (CN)
• Li, Yanfang
Guangdong 510060 (CN)
• Zhou, Taili
Guangdong 510060 (CN)

(72) Inventors:
• Zhang, Yingguang
Guangzhou, Guangdong 510060 (CN)
• Li, Yanfang
Guangdong 510060 (CN)
• Zhou, Taili
Guangdong 510060 (CN)

(74) Representative: Messulam, Alec Moses
A. Messulam & Co. Ltd.
43-45 High Road
Bushey Heath
Hertfordshire WD23 1EE (GB)

(54) X-RAY GENERATOR FOR ACHIEVING STEREOSCOPIC IMAGING EFFECT AND MEDICAL X-RAY DEVICE USING THE X-RAY GENERATOR

(57) The present invention provides an X-ray generator for achieving stereoscopic imaging effect and an X-ray device using the X-ray generator (101) as the X-ray source. The X-ray generator (101) can emit X-rays alternately from two positions the space of which conforms to the requirement for achieving stereoscopic imaging effect of human beings.

FIG. 5

## Description

## Technology Field

The present invention relates to an X-ray generator and an X-ray device, particularly to a medical X-ray generator and a medical X-ray device.

## Background Art

I. X-ray generators in the art

The X-ray tube been in our lives for almost one hundred years, and the X-ray has been applied to various fields of social life, such as industrial radiography, chemical analysis, jewelry appraisal, security inspection in airports and railway stations, X-ray examinations in medical treatment, CT scan device invented in 1970s, Digital Subtraction Angiography, digital X-ray perspective apparatus and Direct Radiography. However, the working principle of the X-ray tube does not change, which is that electron beam bombards heavy metal to generate X-ray.

The earliest X-ray tube is the cold cathode, its working principle is that: an electrode called anode is joined in the discharge path of the discharge tube, and voltage is applied on this anode, the X-ray is generated when the discharge tube discharges. The electrode is made of heavy metal such as molybdenum, tungsten, nickel and cobalt.

The existing and widely used X-ray tubes have two categories: fixing anodes tube and rotating anodes tube.

The tubes which are used in miniature X-ray machines and industrial X-ray machines are fixing anodes tubes, the majority of the packaging mode is a glass tube shell, and a minority is a metal tube shell. The advantages are as follows: the anode is fixed, the structure is simple, and the manufacturing cost is low. The disadvantages are that the power is low and the quantity of X-rays is few so it can only be used in a miniature X-ray machine. A CT machine or a DSA machine cannot apply this kind of tube. The fixing anode tube is comprised of a filament, an anode target which is made of heavy metal and a glass tube shell or metal tube shell which is a pumped vacuum.

Another kind of X-ray tube is a rotating anodes tube. The rotating anodes tube is a high performance tube, which was invented after high power X-ray machines. It can use a higher current to work under small focus. Viewed from the metal heat dissipation, metals all have thermal resistance when they transfer heat, so the anode target may produce a high temperature when the tubes work under small focus and high current. When the electron beam bombards one place continuously, the target may melt quickly. If we make the target move, the electron beam may change the bombardment place, so the tube shell can work under a higher current. The heat which is generated in the target can then transfer into the inner of the metal to prevent melting.

The main difference between the structure of the rotating anodes tube and fixing anodes tube is the anode, the anode of rotating anodes tube is a disk with a shaft, and the anode rotates by rotating magnetic field which is generated by motor stator coil which is mounted outside the tube core. The anode shaft actually is a rotor of hysteresis motor, nothing but it is sealed in vacuum. The function of the rotating anode is that it can generate an X-ray by the bombarding of an electron beam.

The cathode of the rotating is a direct-heating vacuum tube cathode, its function is that it can generate an electron beam to bombard the anode. Its glower is made of tungsten, there is a beam current cover which can control the angle and direction of the electronic ray and it is used to control the size of the focus.

There is a kind of bifocal tube, the kind of bifocal tube has two groups glower cathode, one which can generate big focus has high power and can generate strong X-ray, another one which generate small focus has low power can be used to form more clear image. But there is no difference in position, the big focus covers small focus, a pair of data which accord with the requirements of human stereo vision can't be gained to form stereo vision effect image.

In addition, X-ray tube shell contains circulating cooling system and other assistant system. However, a pair of data which accord with the requirements of human stereo vision can't be gained to form stereo vision effect image.

II. Relief television technology in the art

During the European Renaissance period, the research and practice of painting perspective and sculpture art indicated only provided relatively independent images to the eyes, they can gain genuine stereo vision when the binocular disparity is recovered. In the early stage, binocular stereo vision technology applied the method that observes stereo image by colored spectacles and stereoscope. During the 16th century, people began to apply different colors to protract images which have regular difference for left and right eyes, and then observe the images by filter to generate stereo vision. The stereoscope began being used at the end of 17th century to the early of 18th century, provides an independent visual channel and generates strong stereo vision. This kind of stereoscope is still an efficient means to observe stereo vision. In the 19th century, scientists tried to observe stereo images without using an assistant device but failed.

At the end of the 19th century, people tried to use the film technique to exhibit motive stereo vision. You first adopt two cameras to simulate human binoculars to the screen. The film is then projected to the screen by a bioscope through a polarizing filter. The audience can observe the motive stereo vision by the polarizing filter. The film technique is still used today.

In the early of 20th century, after the appearance of the television technique, people began to develop relief television, the traditional stereo display methods which are used in observing still image or film image. Nine tenths are used in the relief television technique.

In the early period of monochrome television, a more successful technique in relief television combines two television cameras that shoot the images and then transfer the images to two televisions by two independent video channels. A deflection board is mounted on each screen of the television, so this relief television system can gain better deliver stereo images through deflection glasses. This kind of double-channel deflection image separating relief television technique is still a better relief television system.

In the 1950's, color television developed to the practical stage. The anaglyph stereoscopic television technique began to be applied to relief television. The basic method is that two cameras which are mounted filters at the front of the lens are used to screen the same scene image; the audiences can see two images which are different colors from the color television screen. Therefore, audiences could see the relief television image through a corresponding filter. This kind of relief television imaging technique was extremely popular in the relief television technique field because of its good compatibility. However, it had obvious problems: the color information was greatly lost, the cross color caused disturbances, and it could cause visual fatigue because of the inconsistent incident spectral of the left and right eyes.

With the appearance of the ceramic optical switch at the end of 1970's, people could make optical switch glasses, so the time-division relief television technique appeared. The time-division relief television technique adopts the odd field and even a field of color television signal to code relief television. In the early of 1980's, Toshiba Corporation developed a time-division relief television projection machine. In 1985, Panasonic Corporation developed time-division liquid crystal glasses relief television. At present, the helmet watching equipment which has double screens display has very perfect stereo vision effect. Tsinghua University developed the new-type liquid crystal light valve glasses and time-division liquid crystal glasses relief television in 2001.

At present, the time-division relief television technique is mature comparatively. It has obvious advantages like providing lively color stereo image. The image is stabile and non-flickering when the television field frequency is much higher, it is compatible to an actual color television system and computer display system, and the transition to digital television system is easy.

In 2000, the first real time stereo display system appeared in China. Multiple images are played by VCD, so the fierceness stereo image can be gained by wireless infrared glasses. The existing signal source two-dimensional image can be transferred into a three-dimensional image on display by the stereo display system. But this kind of stereo image effect still rests on using the method of optics or signal processing to transfer the image.

The new-type stereo vidicon and stereo display unit are being developed. The new-type stereo vidicon has a double lens, the technology of a comprehensive computer, measurement and control, image treatment, and its shooting process conforms to the requirement for achieving the stereoscopic imaging effect of human beings. The new-type stereo display unit imports left and right images, adopts an optics technique and can project the left and right images to the eyes in accurate parallax. People can therefore watch the stereo image on-screen directly without glasses. In the 3D alliance establishment meeting in Tokyo in 2003, Sanyo showed the display on which the stereo image can be seen without glasses. Sony showed the shooting and playing of the stereo image system. However, the stereo effect of the products above is limited by the observing angle and distance.

The basic principle of relief television technique:

In view of the human vision experience, two eyes observe the vision signals, and the stereo feeling is experienced; people using one eye can also gain stereo feeling by observing the scene and object. The two cases correspond to binocular stereo vision and monocular stereo vision. The former one is the basis of relief television; the latter one is gained by experience.

Binocular stereo vision

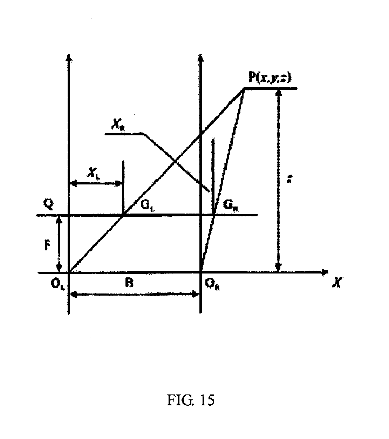

Relief television generates a stereo image by the stereo vision characteristic of human eyes. When humans watch the world around them, they can see not only the width and height of objects, but also the depth, the distance between objects and objects or the viewer. The reason for generating the three-dimension vision characteristic is that people observe objects with two eyes, and the space of the visual axis of two eyes is about 65mm; the left and right eyes receive different images when they watch the object of a certain distance, so the brain generates stereo vision by colligating the information of the two images through the movement and adjustment of eyeball. When people observe objects by the right or left eye the image shift feeling is called parallax. As shown in figure 15.

In figure 15, if there are two identical vidicons, the planes of two images are in the same plane Q, the coordinate axis of two vidicons is parallel, and the X axis is superposition, the space of two vidicons is baseline B in the direction of X. The two projection points $G_L$ and $G_R$ which are projected by the feature point in the scene on the two image planes are called conjugate pairs, that is to say one is the correspondence of another one. After the two images overlap, the position shift $X_L$-$X_R$ of the conjugate pairs is parallax. Suppose the origin of the coordinate system is on the center of left lens, by similar triangle:

$$X/Z=X_L/F \text{ and } (X-B)/Z=X_R/F$$

obtained : $Z=BF/(X_L-X_R)$□

Therefore, the depth of the objects is restored by parallax, the bigger the parallax is, the nearer the distance between object and lens. The stereo image pair is gained by stereo vidicon of the double lens.

The realization mode of relief television

There are mainly two kinds of realization modes of relief television. One kind is two images of a pair of parallax signals appear on the screen at the same time and the stereo vision is gained by seeing the two images, such as dual channel polarizing image separating relief television technique and complementary color stereo image separating television technique. The other kind is two images of a pair of parallax signals appear on the screen alternately, and then the stereo vision is gained by seeing the images at different times, such as the time-division relief television technique.

III. The medical X-ray equipment in existing technique

The medical X-ray diagnosing and treating equipment is made of an X-ray tube of the existing technique, such as X-ray digital subtraction angiography machine, digital stomach and intestines machine, digital X-ray perspective machine, and digital direct radiography system. They all use single X-ray source to form a plane image without stereo feeling.

Moreover, there are double sources CT machine and double C arms DSA machine. The purpose of using double tubes for a double sources CT machine is to improve the scanning speed not to form stereo vision; the purpose of using double tubes for double C arms DSA machine is to add a projective angle, because without fixed ubiety for double C arms, the two images cannot form a pair of data which conform to the requirement for achieving stereoscopic imaging effect of human beings to generate a stereo effect image.

With the development of modern medicine, an understanding of the spatial position and relation of human tissues and structure is necessary. Therefore the three-dimensional reconstruction techniques based on computer technology were developed such as CT three-dimension reconstruction technique, MRA three-dimension reconstruction technique, and DSA three-dimension reconstruction technique. However, these three-dimensional reconstruction techniques cannot provide real time dynamic stereo images so doctors only rely on a single point X-ray source which is provided by the existing X-ray tube and operates through a plane image. In this situation, doctors can only differentiate up and down or left and right, but cannot differentiate forward and backward. This creates a risk for interventional procedures, especially neural interventional, cardiac interventional and peripheral interventional.

As a conclusion, the X-ray tubes in existing technology are very mature and exact, but they can only provide single source and form plane image; they cannot provide a pair of data which conform to the requirement for achieving stereoscopic imaging effect of human beings and form stereo vision effect. Although the medical X-ray equipment is also mature and exact, it cannot form real time dynamic stereo vision effect images.

Obviously, the existing X-ray tubes cannot achieve this objective, and developing a new X-ray generator which can provide real time dynamic stereo vision effect is a useful endeavor. This kind of medical X-ray equipment can generate stereo vision. In the perspective mode, a physician performing an invasive procedure could see as 3-dimensional the skull, blood vessel, and bones, thereby lowering the risk of an operation.

## Contents of the Invention

The present invention provides an X-ray generator for achieving stereoscopic imaging effect and a medical X-ray device for achieving stereoscopic imaging effect using the X-ray generator as key parts.

The purpose of the present invention is realized as follows:

An X-ray generator for achieving stereoscopic imaging effect and an X-ray device with stereoscopic imaging effect are provided, the X-ray generator deployed to emit X-ray alternately from two positions the space of which conforms to the requirement for achieving stereoscopic imaging effect of the human beings, and the X-ray device uses the X-ray generator as the X-ray source.

According to the further feature of the invention, the space of the two positions is close to papillary distance and the space is 40 mm to 90 mm, and the optimum space is 58 mm to 72 mm. The space of the two positions may be fixed or could be adjusted.

According to the further feature of the invention, the X-ray generator comprises at least two X-ray tubes (1) which emit X-ray alternately, and the space (D) of the X-ray tubes (1) is 40 mm to 90 mm, and the optimum space is 58 mm to 72 mm.

According to the further feature of the invention, the X-ray generator for achieving stereoscopic imaging effect comprises two X-ray tubes, thus could be called two-tube type stereo vision X-ray generator. The X-ray generator for achieving stereoscopic imaging effect comprises three X-ray tubes, thus could be called three-tube type stereo vision X-ray generator.

However, no matter how many X-ray tubes are applied, only when the space of the two tubes which emit X-ray alternately accord with the requirements of human stereo vision, a pair of data which accord with the requirements of human stereo vision can be gained, and is independent and associated, and relief television technique or vector-graph technique is applied to form stereoscopic imaging effect.

The X-ray generator for achieving stereoscopic imaging effect comprises cathode, anode, deflection electrode, and at least two anode focuses; the high energy electron beam which is emitted from cathode and controlled by deflection electrode hits the different anode focuses to emit X-ray alternately. The space of different anode focuses is 40 mm to 90 mm; the optimum space is 58 mm to 72 mm. The type of X-ray generators for achieving stereoscopic imaging effect in the present invention can be designed into many different detailed structures, for example, in accordance with the number of anode focus, they can be divided into single-focus type, double-focus type, three-focus type and so on; in accordance with the number of anode, they can be divided into single-anode type, double-anode type, three-anode type and so on; in accordance with anode moving or not, they can be divided into fixing anodes and rotating anodes.

However, no matter how many X-ray tubes are applied, only when the space of the two tubes which emit X-ray alternately accord with the requirements of human stereo vision, a pair of data which accord with the requirements of human stereo vision can be gained, and is independent and associated, and relief television technique or vectorgraph technique is applied to form stereoscopic imaging effect.

Further, the X-ray generators for achieving stereoscopic imaging effect have three anode focuses, which are deployed to emit X-ray alternately from two or three anode focuses. When the space of any two focuses of one-tube and three-focus X-ray generators for achieving stereoscopic imaging effect accords with the requirements of human stereo vision, data which accords with the requirements of human stereo vision can be gained and forms stereoscopic imaging effect. In this way, one-tube and three-focus X-ray generators for achieving stereoscopic imaging effect can provide three different angles stereo vision image without moving the X-ray generators.

The X-ray generators for achieving stereoscopic imaging effect have at least two cathodes, the high energy electron beam from each cathode hits the corresponding anode focuses alternately, and the X-ray is emitted from anode focus alternately. In accordance with the number of cathodes, they can be divided into single-cathode type, double- cathode type, three- cathode type and so on.

The present invention also provides X-ray device which applies X-ray generators for achieving stereoscopic imaging effect, especially medical X-ray device for achieving stereoscopic imaging effect.

The medical X-ray device for achieving stereoscopic imaging effect applies X-ray generator for achieving stereoscopic imaging effect as the X-ray source.

Further, the medical X-ray device for achieving stereoscopic imaging effect includes a digital subtraction angiography for achieving stereoscopic imaging effect, which applies X-ray generator for achieving stereoscopic imaging effect as the X-ray source for digital subtraction angiography.

The medical X-ray device for achieving stereoscopic imaging effect includes a digital gastrointestinal apparatus for achieving stereoscopic imaging effect, which applies X-ray generator for achieving stereoscopic imaging effect as the X-ray source for digital gastrointestinal apparatus.

The medical X-ray device for achieving stereoscopic imaging effect includes a digital X-ray perspective apparatus for achieving stereoscopic imaging effect, which applies X-ray generator for achieving stereoscopic imaging effect as the X-ray source for digital X-ray perspective apparatus.

The medical X-ray device for achieving stereoscopic imaging effect includes a direct radiography (DR) for achieving stereoscopic imaging effect, which applies X-ray generator for achieving stereoscopic imaging effect as the X-ray source for digital direct radiography.

The X-ray generator for achieving stereoscopic imaging effect is not only used as the X-ray source of medical X-ray generator for achieving stereoscopic imaging effect, but can also be used in industrial radiography, jewelry appraisal, security inspection in airports and railway stations, and for X-ray examinations in medical treatment

The present invention provides an X-ray generator for achieving stereoscopic imaging effect and an X-ray device using the X-ray generator as the X-ray source. The X-ray generator can emit X-rays alternately from two positions the space of which conforms to the requirement for achieving stereoscopic imaging effect of human beings, so a pair of X-ray image data which conforms to the requirement for achieving stereoscopic imaging effect of the human beings can be gained. The pair of data is treated by a computer data processing system to form a stereo image in stereo image display system by relief television technique and stereo film technique. By the medical X-ray generator for achieving stereoscopic imaging effect in the present invention doctors can observe real time dynamic stereo vision effect image which is very convenience for doctors in diagnosing and operating.

**Brief Description of the Figures**

Figure 1 is a perspective view illustrating the structure of the double-tube X-ray generator for achieving stereoscopic imaging effect;

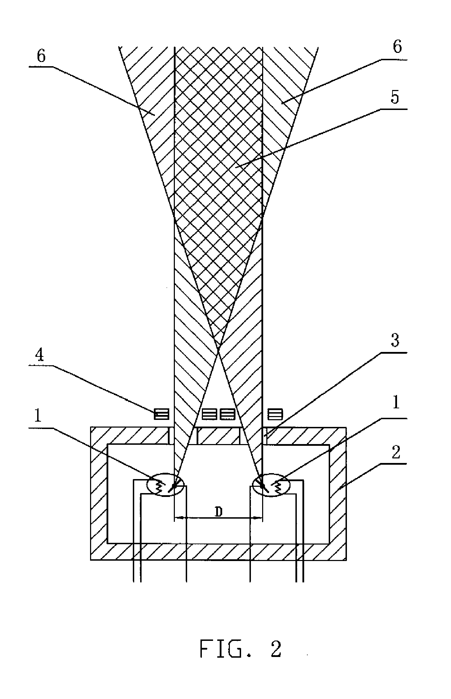

Figure 2 is a perspective view illustrating the structure of double-tube and double-window type X-ray generator for achieving stereoscopic imaging effect. The difference from figure 1 is that every X-ray tube emits X-ray from the corresponding window;

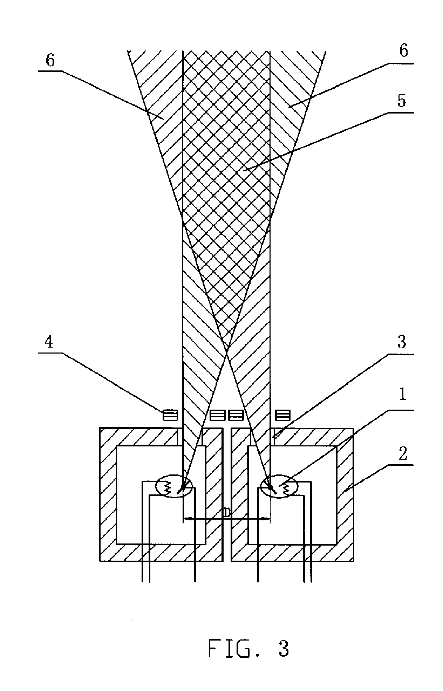

Figure 3 is a perspective view illustrating the structure of the independent double-tube type X-ray generator for achieving stereoscopic imaging effect. The difference from figure 2 is that every X-ray tube which the shielding case shields emits X-ray from corresponding window;

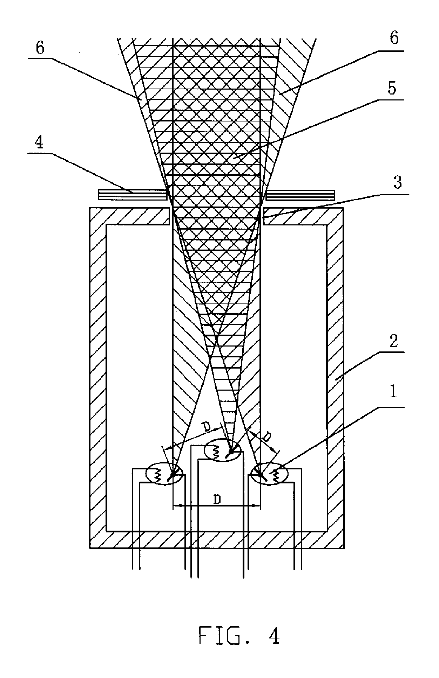

Figure 4 is a perspective view illustrating the structure of the three-tube X-ray generator for achieving stereoscopic imaging effect. The difference among figures 1-3 is that this kind of generator has one more X-ray tube and a total of three tubes. Therefore, three different angle stereo vision images can be gain without rotating the X-ray generator;

Figure 5 is a perspective view illustrating the structure of a single-tube and double-focus type X-ray generator for achieving stereoscopic imaging effect;

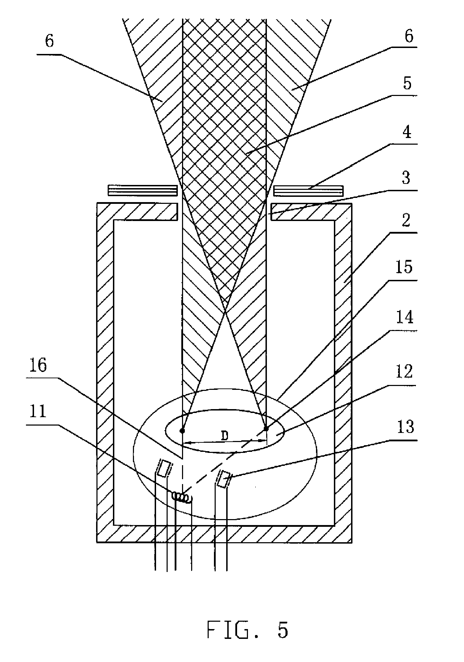

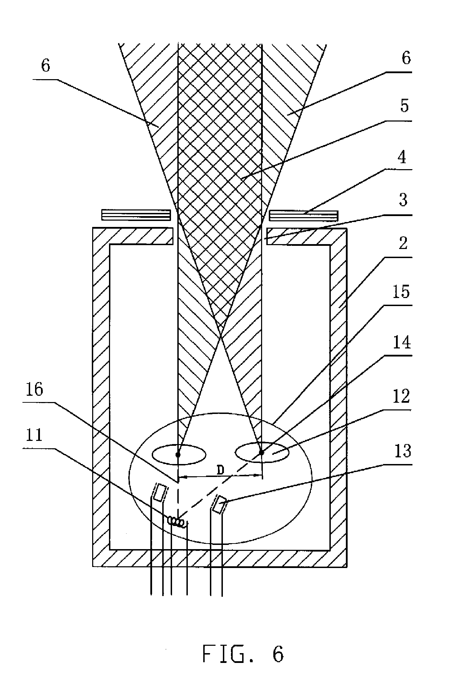

Figure 6 is a perspective view illustrating the structure of a single-tube, double-focus and double-anode type X-ray generator for achieving stereoscopic imaging effect. The difference between Figure 6 and Figure 5 is that Figure 6 adopts two rotating anodes. A high energy electron beam from the cathode bombards two rotating anodes alternately to form two anode focuses and future form two X-ray source. The structure in figure 5 has a single rotating anode, a high energy electron beam from the cathode bombards two fixing positions in the same rotating anodes alternately to form two anode focuses in the same rotating anodes and future form two X-ray sources;

Figure 7 is a perspective view illustrating the structure of a single-tube, three-focus type X-ray generator for achieving stereoscopic imaging effect. The difference between Figure 7 and Figure 5 is that Figure 7 adopts three focuses, a high energy electron beam from the cathode bombards three fixing positions in the same rotating anodes alternately to form three anode focuses in the same rotating anodes and future form three X-ray sources. Data from any two focuses can form stereo vision image, so three different angle stereo vision images can be gained without rotating the X-ray generator;

Figure 8 is a perspective view illustrating the structure of a single-tube, two-focus and double-cathode type X-ray generator for achieving stereoscopic imaging effect. The difference between Figure 8 and Figure 5 is that Figure 8 has two cathodes; the high energy electron beam from two cathodes bombards the corresponding positions in the rotating anode to form two anode focuses in the same rotating anode and future form two X-ray sources;

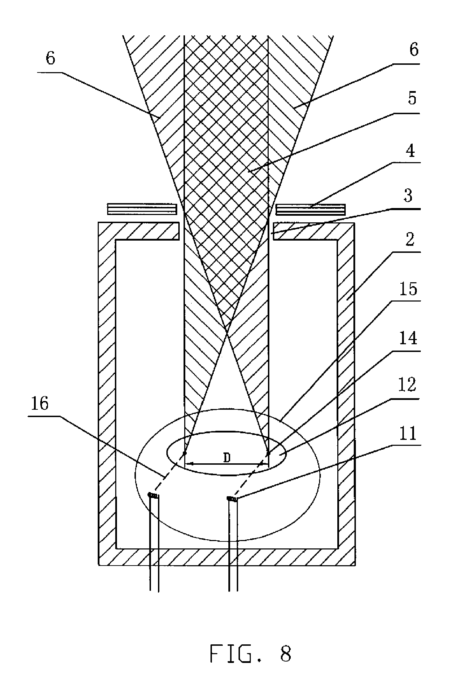

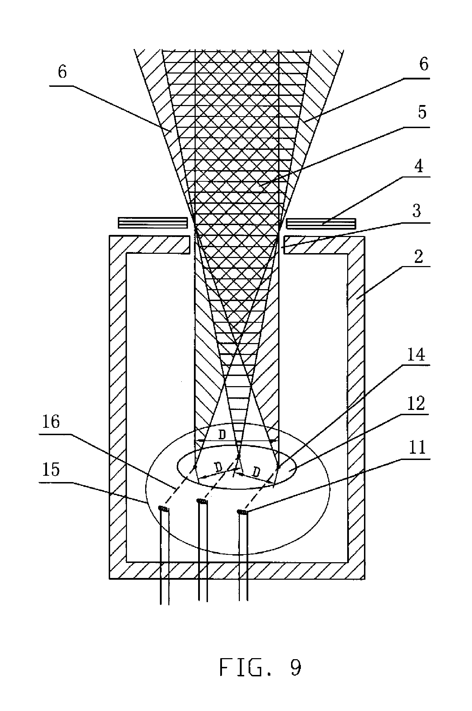

Figure 9 is a perspective view illustrating the structure of a single-tube, three-focus and three-cathode type X-ray generator for achieving stereoscopic imaging effect. The difference between Figure 9 and Figure 8 is that Figure 9 adopts three cathodes, a high energy electron beam from three cathodes bombards three fixing positions in the same rotating anodes alternately to form three anode focuses in the same rotating anodes and future form three X-ray sources. Data from any two focuses can form stereo vision image, so three different angle stereo vision images can be gained without rotating the X-ray generator;

Figure 10 is a perspective view illustrating the working principle of real time dynamic stereo vision image diagnoses interventional therapy apparatus which installs a single-tube and two-focus type X-ray generator for achieving stereoscopic imaging effect;

Figure 11 is a perspective view illustrating the working principle of real time dynamic stereo vision image diagnoses interventional therapy apparatus which installs single-tube and two-focus type X-ray generator for achieving stereoscopic imaging effect. The difference between Figure 11 and Figure 10 is that in Figure 11, doctors can observe stereo vision image directly on the display of the stereo vision display system without stereo vision glasses;

Figure 12 is a perspective view illustrating the working principle of real time dynamic stereo vision image digital X-ray perspective apparatus which install single-tube and three-focus type X-ray generator for achieving stereoscopic imaging effect;

Figure 13 is a perspective view illustrating the working principle of X-ray real time dynamic stereo vision digital intestines and stomach machine which installs a double-tube type X-ray generator for achieving stereoscopic imaging effect;

Figure 14 is a perspective view illustrating the working principle of X-ray real time dynamic stereo vision image direct radiography machine which installs a three-tube type X-ray generator for achieving stereoscopic imaging effect; and

Figure 15 is a perspective view illustrating two-eye type stereo vision.

In these figures, 1 denotes the X-ray tube, 2 denotes the shielding case, 3 denotes the window, 4 denotes the grating, 5 denotes the stereovision data acquisition area, 6 denotes the non-stereovision data acquisition area, 11 denotes the cathode, 12 denotes the anode, 13 denotes the deflection electrode, 14 denotes the anode focus, 15 denotes the vacuum case, 16 denotes the high energy electron beam, 101 denotes the X-ray generator for achieving stereoscopic imaging effect, 111 denotes the data acquisition system, 112 denotes the computer data processing system, 113 denotes the stereo image display system, 114 denotes the manipulator, 115 denotes the electrical source and assistant equipment, 116 denotes the patient, 117 denotes the operation table, 118 denotes the physician, 119 denotes the stereo vision glasses.

## Detailed Description of the Invention

Example 1: Double-tube type X-ray device for achieving stereoscopic imaging effect

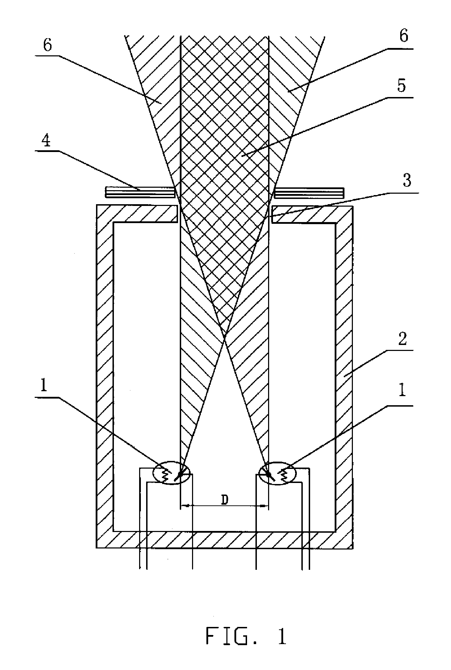

Refer to Figure 1, in this example, two X-ray tubes 1 may adopt rotating anode tube, the space between the anode focus of the two tubes keeps D, space D is from 40mm to 90mm, the optimum value is 58 mm to 72 mm. The two X-ray tubes 1 are mounted in shielding case 2. X-ray window 3 is mounted on shielding case 2, grating 4 controls the size of the X-ray beam.

With the help of an electrical source and assistant equipment, the electron beam from cathode 11 which is controlled by deflection electrode 13 bombards anode focus 14 to emit X-ray on two anode focuses alternately. The region which is on the outside of the shielding case 2 and covered by the X-ray beam which is emitted from two focuses 14 is stereovision data acquisition area 5, and the region which is covered by the X-ray beam which is emitted from only one focus 14 is non-stereovision data acquisition area 6.

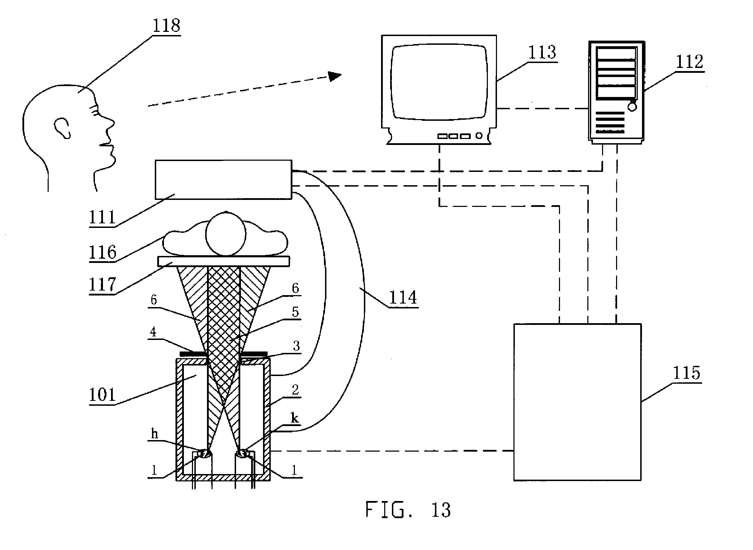

As shown in figure 13, as a matter of convenience, X-ray tube 1 in the left of the double-tube type X-ray device for achieving stereoscopic imaging effect 101 is marked h, the right X-ray tube 1 is marked k, the space between the anode focus of tube h and the anode focus of tube k is marked D. The size of space D may be controlled by an electromechanical device; the value is from 40 mm to 90 mm and usually is 65 mm $\pm$ 2 mm.

The X-ray which is emitted from the double-tube type X-ray device for achieving stereoscopic imaging effect 101 is controlled by grating 4 to adjust the section which is covered by the X-ray beam and control imaging view. The X-ray passes through the operation table 117 and patient 116, and then shoots the data acquisition system 111, the data acquisition system 111 is mounted on the section which is covered by the X-ray beam and used to acquire image data.

X-ray data acquisition system 111 marks the data which is formed by the X-ray beam emitted from tube h as H data team and the data which is formed by the X-ray beam emitted from tube k as K data team thereinto, the data which is formed by the X-ray beam emitted from tube h and noted in stereovision data acquisition area 5 is marked H5 and the data which is formed by the X-ray beam emitted from tube k and noted in stereovision data acquisition area 5 is marked K5. Data H5 and K5 are associated data which conform to the requirement for achieving stereoscopic imaging effect of human beings, data H5 and K5 which are treated by the computer data processing system form the stereo image in stereo image display system 113 by the relief television technique and the stereo film technique.

There are many manners in which to form the stereo image in stereo image display system 113. There are mainly two types of mature technologies. One kind is that two images of a pair of parallax signals appear on the screen at the same time, and then the stereo vision is gained by seeing the two images, such as dual channel polarizing image separating relief television technique and complementary color stereo image separating television technique. Another kind is that two images of a pair of parallax signals appear on the screen alternately, and then the stereo vision is gained by seeing the images at different time, such as time-division relief television technique. Furthermore, the new stereo display unit may also be applied. It can import left and right images at the same or different times and adopt optical technology to make the left and right image project onto the eyes, so the stereo image can be seen on the screen without glasses.

Electrical source and assistant equipment 115 can provide power for the whole machine and control automation. For example, the movement of manipulator 114 can gain real time stereo vision imaging effect of different angles and different position, which makes operation convenient for doctor 118.

Example 2: Single-tube and double-focus type X-ray device for achieving stereoscopic imaging effect

As shown in figure 5, this example is very different from example 1.

In this example, the single-tube and double-focus type X-ray device for achieving stereoscopic imaging effect is adopted. Its basic structure principle has the similar part to the routine rotating anode tube in the existing technique, comprising cathode 11 and anode 12, but it has a deflection electrode 13 which can control the directional of the cathode electron beam. The electron beam from cathode 11 which is be controlled by deflection electrode 13 bombards focus 14 on anode 12 to emit X-ray alternately.

The space between two focuses 14 keeps D, the value of D is from 40mm to 90mm, the optimal value is from 58mm to 72mm. The position of anode focus 14 on anode 12 can be adjusted by adjusting deflection electrode 13, and the space D between two focuses 14 is controlled at 65 mm $\pm$ 2 mm.

Cathode 11, anode 12 and deflection electrode 13 are set inside the vacuum case 15, and then the vacuum case is set inside the shielding case 2. The X-ray window 3 is mounted on the shielding case 2, and the size of the X-ray beam is controlled by grating 4.

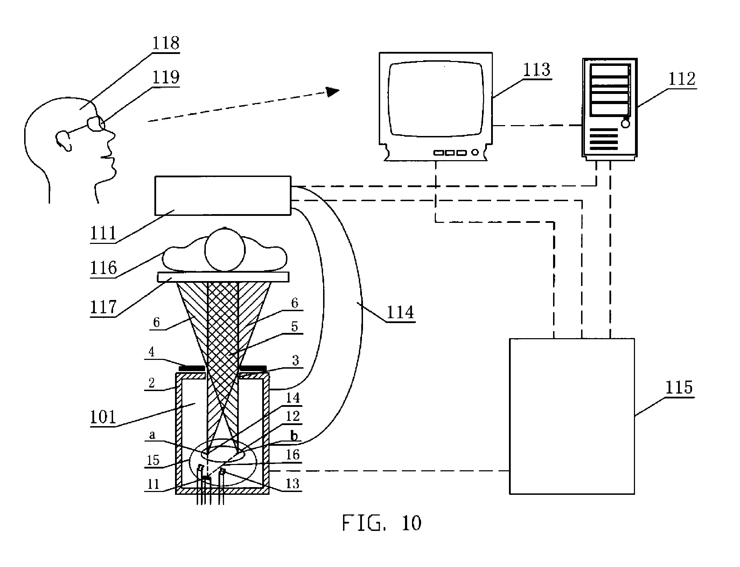

As shown in figure 10, with the help of an electrical source and assistant equipment, the electron beam from cathode 11 which is controlled by deflection electrode 13 bombards anode focus 14 to emit X-ray on two anode focuses alternately. The region which is on outside shielding case 2 and covered by the X-ray beam which is emitted from two focuses 14 is stereovision data acquisition area 5, and the region which is covered by the X-ray beam which is emitted from only one focus 14 is non-stereovision data acquisition area 6.

X-ray data acquisition system 111 is installed on the region which is covered by X-ray beam, the data which is formed by X-ray which is emitted from the same focus in stereovision data acquisition area 5 is noted as one team. For clear expression, the left anode focus is marked as a, and the right anode focus is marked as b, the data which is formed by the X-ray beam emitted from focus a and noted in stereovision data acquisition area 5 is marked A5 and the data which is formed by X-ray beam emitted from focus b and noted in stereovision data acquisition area 5 as B5. Data A5 and B5 are associated data which conforms to the requirement for achieving stereoscopic imaging effect of human beings, data A5 and B5 which are treated by computer data processing system 112 form stereo image in stereo image display system 113 by relief television technique and stereo film technique.

Example 3: Single-tube and three-focus type X-ray device for achieving stereoscopic imaging effect

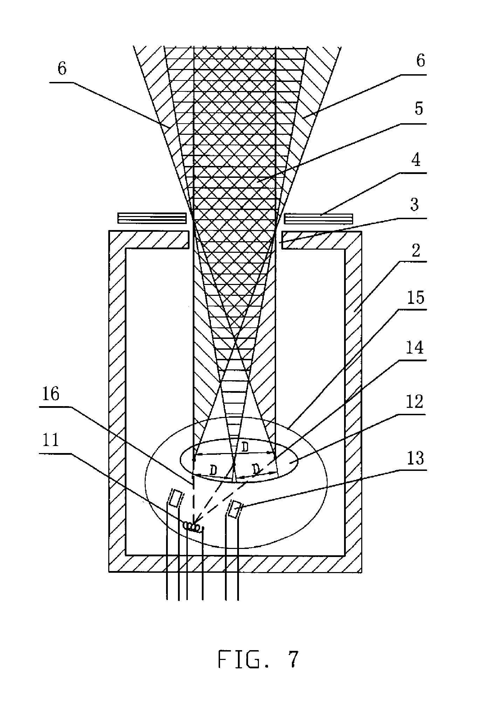

As is shown in figure 7, the basic principle in this example is similar to example 2, comparing figure 7 and figure 5. Different from example 2, it adopts three focuses, the high energy electron beam 16 from cathode 11 bombards three different position on the same rotating anode 12 alternately, forming three anode focuses 14 on single rotating anode, and the X-ray is emitted from the three anode focuses 14 alternately.

If the space of any two of the three focuses all conforms to the requirement for achieving stereoscopic imaging effect of human beings, three pairs of data which can be gained conform to the requirement for achieving stereoscopic imaging effect of human beings, and corresponding three pairs of stereo vision image can be formed. Three different angles of stereo vision image can be gained by this kind of technique without rotating the X-ray generator. If only the doctors switch the images, they can observe the real time stereo dynamic images from three different angles, which is very convenient for diagnosing and operation.

Example 4: Single-tube, double-cathode and double-focus type X-ray device for achieving stereoscopic imaging effect

As is shown in figure 8, the basic principle in this example is similar to example 2, comparing figure 8 to figure 5.

Different from Example 2, Example 4 adopts two cathode 11, of which high energy electron beam 16 bombards corresponding position on the same rotating anode 12. Two anode focuses 14 are formed on the same rotating anode 12 and two X-ray sources are formed. A pair of data which conforms to the requirement for achieving stereoscopic imaging effect of human beings is provided to form stereo vision image.

Example 5: Single-tube, three-cathode and three-focus type X-ray device for achieving stereoscopic imaging effect

As is shown in figure 9, the basic principle in this example is similar to example 4, comparing figure8 to figure 9

Different from example 4, example 5 adopts three cathodes 11, the high energy electron beam 16 from three cathodes 11 bombards corresponding position on the same rotating anode 12, forming three anode focuses 14 on single rotating anode, and three X-ray sources. If the space of any two of the three focuses all conforms to the requirement for achieving stereoscopic imaging effect of the human beings, three pairs of data which can be gained conforms to the requirement for achieving stereoscopic imaging effect of human beings, and corresponding three pairs of stereo vision image can be formed. Three different angles of stereo vision image can be gained by this kind of technique without rotating the X-ray generator. If only the doctors switch the images, they can observe the real time stereo dynamic images from three different angles, which is very convenience for diagnosing and operation.

Example 6: Real time dynamic stereo vision image diagnose interventional therapy apparatus

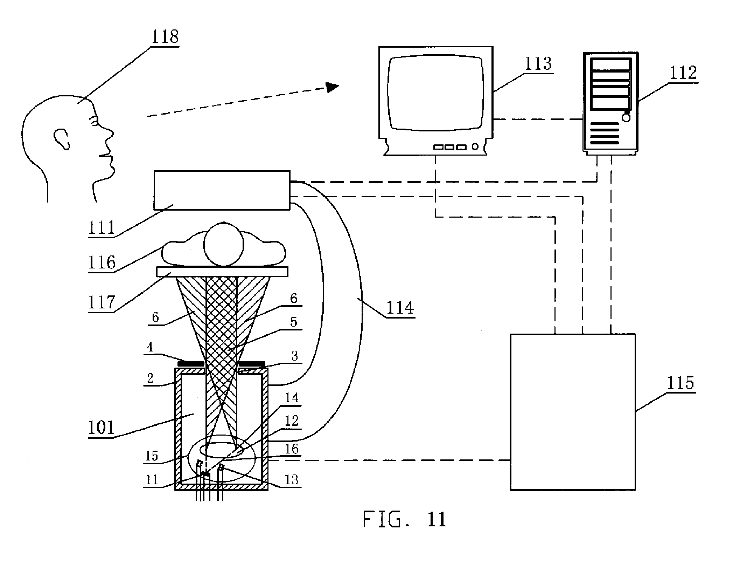

As shown in figures 10 and 11, in this example, the single-tube and double-focus type X-ray device for achieving stereoscopic imaging effect is used as the X-ray source of real time dynamic stereo vision image diagnose interventional therapy apparatus.

With the help of an electrical source and assistant equipment, the electron beam from cathode 11 which is controlled by deflection electrode 13 bombards anode focus 14 to emit X-ray on two anode focuses alternately. A pair of date which conforms to the requirement for achieving stereoscopic imaging effect of the human beings is gained by X-ray data acquisition system 111 in stereovision data acquisition area 5. The pair of data is treated by computer data processing system 112 to form a stereo image in stereo image display system 113 by the relief television technique and stereo film technique.

This kind of medical X-ray equipment can generate stereo vision. In the perspective mode, the interventional physician can see the three-dimensional skull as crystal, blood vessel and bones and the risks during an operation are lessened.

In this example, the relief television technique and stereo film technique, such as time-division relief television technique, consists of a dual channel polarizing image separating relief television technique and complementary color stereo image separating television technique. The doctors 118 need stereo glasses 113 to observe the stereo vision image in the stereo image display system. See figure 10.

The new stereo display unit may also be applied. It can import left and right images at the same or different times and adopts optical technology to make the left and right image project onto eyes, so the stereo image can be seen on the screen without glasses. See figure 11.

Example 7: Real time dynamic stereo vision image digital X-ray perspective apparatus

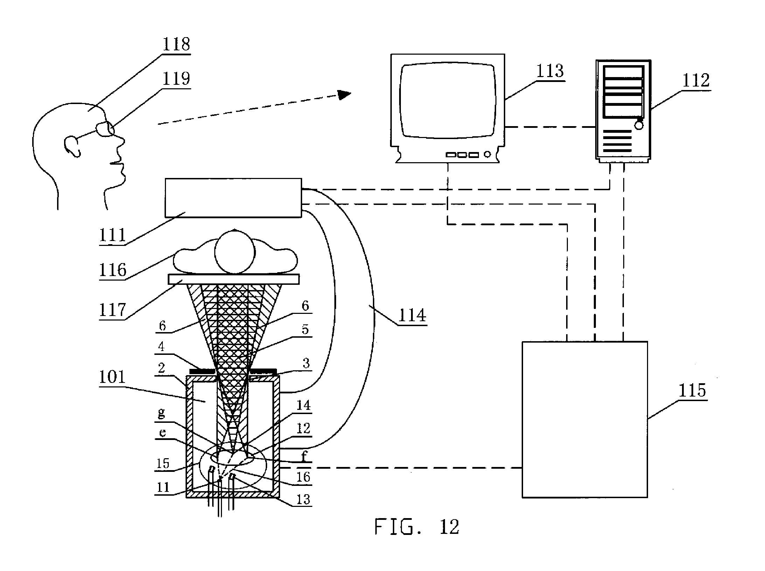

As is shown in figure 12, in this example, the single-tube and three-focus type X-ray device for achieving stereoscopic imaging effect is used as the X-ray source of real time dynamic stereo vision image digital X-ray perspective apparatus.

With the help of an electrical source and assistant equipment, the high energy electron beam 16 from cathode 11 bombards three corresponding positions on the same rotating anode 12 alternately, forming three anode focuses 14 on single rotating anode and three X-ray sources.

If the space of any two of the three focuses all conforms to the requirement for achieving stereoscopic imaging effect of the human beings, three pairs of data which can be gained conform to the requirement for achieving stereoscopic imaging effect of human beings, and corresponding three pairs of stereo vision image can be formed. Three different angles of stereo vision image can be gained by this kind of technique without rotating the X-ray generator. If only the doctors switch the images, they can observe the real time stereo dynamic images from three different angles. In the perspective mode, the surgical doctor can see human tissues which have a third dimension. With this equipment, the diagnosis is more exact. See figure 12.

Example 8: X-ray real time dynamic stereo vision digital intestines and stomach machine.

As is shown in figure 13, in this example, the double-tube type X-ray device for achieving stereoscopic imaging effect is used as the X-ray source of X-ray real time dynamic stereo vision digital intestines and stomach machine.

With the help of an electrical source and assistant equipment, the electron beam from cathode 11 which is controlled by deflection electrode 13 bombards anode focus 14 to emit the X-ray on two anode focuses alternately. The region which is on the outside of the shielding case 2 and covered by the X-ray beam which is emitted from two focuses 14 is stereovision data acquisition area 5, and the region which is covered by the X-ray beam which is emitted from only one focus 14 is non-stereovision data acquisition area 6.

X-ray tube 1 in the left of the double-tube type X-ray device for achieving stereoscopic imaging effect 101 is marked h, the right X-ray tube 1 is marked k, and the space between the anode focus of tube h and the anode focus of tube k is marked D. The size of the space D may be controlled by an electromechanical device; the value is from 40 mm to 90 mm and usually is 65 mm ± 2 mm.

The size of the X-ray emitted from the double-tube type X-ray device for achieving stereoscopic imaging effect 101 is controlled by grating 4, thereby the region covered by the X-ray is regulated, and forms the visual field. The X-ray goes through the operation table 117 and the patient 116, and goes to the data acquisition system 111, and the data acquisition system 111 receives the image data from the region covered by the X-ray.

The X-ray data acquisition system 111 marks the data which is formed by the X-ray beam emitted from tube h as H data team and the data which is formed by the X-ray beam emitted from tube k as K data team, thereinto, the data which is formed by the X-ray beam emitted from tube h and noted in stereovision data acquisition area 5 is marked as H5 and the data which is formed by the X-ray beam emitted from tube k and noted in stereovision data acquisition area 5 is marked K5. Data H5 and K5 are associated data which conforms to the requirement for achieving stereoscopic imaging effect of human beings, data H5 and K5 are treated by the computer data processing system to form the stereo image in stereo image display system 113 by relief television technique and stereo film technique.

The electrical source and assistant equipment 115 can provide power for the whole machine and automated control. For example, the movement of manipulator 114 can gain real time stereo vision imaging effect of different angles and different position, which makes operation convenient for doctor 118.

Example 9: X-ray real time dynamic stereo vision image direct radiography (DR) machine.

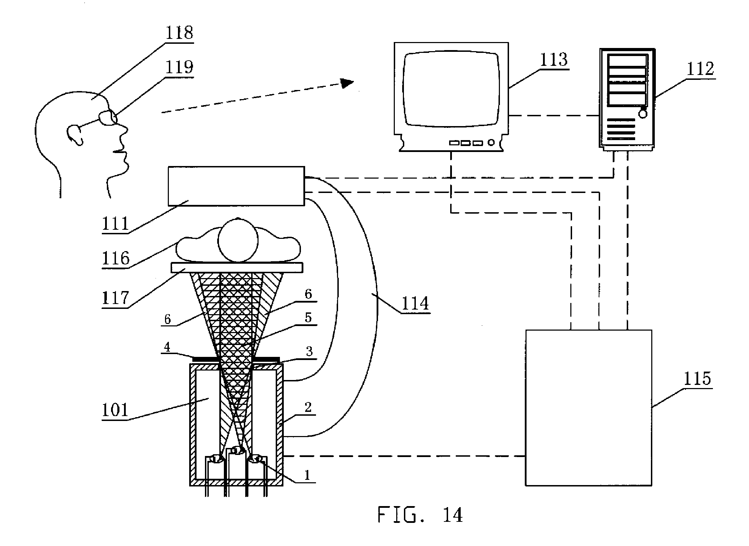

As is shown in figure 14, in this example, the three-tube type X-ray device for achieving stereoscopic imaging effect is used as the X-ray source of X-ray real time dynamic stereo vision image direct radiography machine.

With the help of an electrical source and assistant equipment, the three X-ray tubes emit X-ray alternately; a pair of data which conforms to the requirement for achieving stereoscopic imaging effect of human beings is gained by X-ray data acquisition system 111. The pair of data is treated by computer data processing system 112 to form stereo image in stereo image display system 113 by relief television technique and stereo film technique. Three different angles of stereo vision image can be gained by this kind of technique without rotating the X-ray generator. If the doctors switch the images, they can observe the real time stereo dynamic images from three different angles. In the perspective mode, the surgical doctor can see human tissue which has a third dimension. With this equipment, the diagnosis is more exact.

## Claims

1. An X-ray generator (101) for achieving stereoscopic imaging effect and an X-ray device with stereoscopic imaging effect, **characterized by** the X-ray generator being deployed to emit X-rays alternately from two positions the space of which conforms to the requirement for achieving stereoscopic imaging effect of human beings, and the X-ray device uses the X-ray generator (101) as the X-ray source.

2. The X-ray generator for achieving stereoscopic imaging effect according to claim 1, wherein the space of the two positions is close to papillary distance and the space is 40 mm to 90 mm, and the optimum space is 58 mm to 72 mm.

3. The X-ray generator for achieving stereoscopic imaging effect according to claim 1, wherein said X-ray generator comprises at least two X-ray tubes (1) which emit X-rays alternately, and the space (D) of the X-ray tubes (1) is 40 mm to 90 mm, and the optimum space is 58 mm to 72 mm.

4. The X-ray generator for achieving stereoscopic imaging effect according to claim 1, wherein said X-ray generator comprises cathode (11), anode (12), deflection electrode (13), and at least two anode focuses (14); the high energy electron beam emitted from cathode (11) and controlled by deflection electrode (13) hits the different anode focuses (14) to emit X-ray alternately; the space (D) of different anode focuses (14) is 40 mm to 90 mm, and the optimum space (D) is 58 mm to 72mm.

5. The X-ray generator for achieving stereoscopic imaging effect according to claim 4, wherein said X-ray generator has three anode focuses (14), deployed to emit X-ray alternately from two or three anode focuses (14).

6. The X-ray generator for achieving stereoscopic imaging effect according to claim 4, wherein said X-ray generator has at least two cathodes (11), the high energy electron beam from each cathode (11) hits the corresponding anode focuses (14) alternately, and the X-ray is emitted from anode focus (14) alternately.

7. The stereoscopic imaging effect medical X-ray device according to claim 1, comprising a digital subtraction angiography for achieving stereoscopic imaging effect, which applies X-ray generator for achieving stereoscopic imaging effect as the X-ray source for digital subtraction angiography.

8. The stereoscopic imaging effect medical X-ray device according to claim 1, comprising a digital gastrointestinal apparatus for achieving stereoscopic imaging effect, which applies X-ray generator for achieving stereoscopic imaging effect as the X-ray source for digital gastrointestinal apparatus.

9. The stereoscopic imaging effect medical X-ray device according to claim 1, comprising real time dynamic stereo vision image digital X-ray perspective apparatus, wherein the real time dynamic stereo vision image digital X-ray perspective apparatus uses the X-ray generator for achieving stereoscopic imaging effect as X-ray source.

10. The stereoscopic imaging effect medical X-ray device according to claim 1, comprising a digital X-ray perspective apparatus for achieving stereoscopic imaging effect, which applies X-ray generator for achieving stereoscopic imaging effect as the X-ray source for digital X-ray perspective apparatus.

6  6

5

4

3

2

1  1

D

FIG. 1

FIG. 2

FIG. 3

FIG. 4

FIG. 5

FIG. 6

FIG. 7

FIG. 8

FIG. 9

FIG. 10

FIG. 11

FIG. 12

EP 2 135 553 A1

FIG. 13

FIG. 14

FIG. 15

# INTERNATIONAL SEARCH REPORT

| | International application No. |
|---|---|
| | PCT/CN2008/070664 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)

IPC: A61B6, G03B42

Documentation searched other than minimum documentation to the extent that such documents are included in the fields searched

Electronic data base consulted during the international search (name of data base and, where practicable, search terms used)

WPI,EPODOC,PAJ,CNPAT stereo+,space+,distance, x w ray?, xray?

**C. DOCUMENTS CONSIDERED TO BE RELEVANT**

| Category* | Citation of document, with indication, where appropriate, of the relevant passages | Relevant to claim No. |
|---|---|---|
| X | US2006/0227936A1 ( DONG Z et al ) 12 Oct.2006(12.10.2006), Description paragraphs [0005],[0009],[0020]-[0021], figures 1-2 | 1-3, 7-10 |
| X | US2002/0126796A1 (YAMASAKI T) 12 Sep.2002(12.09.2002), Description paragraphs [0014]-[0025], figure 1 | 1-3, 7-10 |
| X | US4413352A (TOKYO SHIBAURA DENKI K) 01 Nov.1983 (01.11.1983), Description column 2 line 52 to column 3 line 29, figure 1 | 1-2, 7-10 |
| X | US4819255A (TOSHIBA KK) 04 Apr. 1989 (04.04.1989), Description column 3 line 21 to line 63, figure 3 | 1-2, 7-10 |
| X | JP2005168601A (CANON KK ) 30 Jun. 2005(30.06.2005), Description paragraphs [0013]-[0039], figure 1 | 1-3, 7-10 |
| E | CN201018708Y ( ZHANG, Yingguang ) 06 Feb. 2008 (06.02.2008), the whole document | 1-10 |

☐ Further documents are listed in the continuation of Box C.  ☒ See patent family annex.

| | |
|---|---|
| *  Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim (S) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"document member of the same patent family |

| Date of the actual completion of the international search | Date of mailing of the international search report |
|---|---|
| 11 Jun. 2008 (11.06.2008) | **17 Jul. 2008 (17.07.2008)** |

| Name and mailing address of the ISA/CN | Authorized officer |
|---|---|
| The State Intellectual Property Office, the P.R.China<br>6 Xitucheng Rd., Jimen Bridge, Haidian District, Beijing, China 100088 | XU,Min |
| Facsimile No. 86-10-62019451 | Telephone No. (86-10)62085623 |

Form PCT/ISA/210 (second sheet) (April 2007)

<table>
<tr><td colspan="2"><b>INTERNATIONAL SEARCH REPORT</b><br>Information on patent family members</td><td colspan="2">International application No.<br><br>PCT/CN2008/070664</td></tr>
<tr><td>Patent Documents referred<br>in the Report</td><td>Publication Date</td><td>Patent Family</td><td>Publication Date</td></tr>
<tr><td>US2006/0227936A1</td><td>12.10.2006</td><td>None</td><td></td></tr>
<tr><td>US2002/0126796A1</td><td>12.09.2002</td><td>JP2002263091A</td><td>17.09.2002</td></tr>
<tr><td></td><td></td><td>US6449333B1</td><td>10.09.2002</td></tr>
<tr><td>US4413352A</td><td>01.11.1983</td><td>EP0051430A</td><td>12.05.1982</td></tr>
<tr><td></td><td></td><td>JP57076800A</td><td>13.05.1982</td></tr>
<tr><td></td><td></td><td>EP0051430B</td><td>24.07.1985</td></tr>
<tr><td></td><td></td><td>DE3171503G</td><td>29.08.1985</td></tr>
<tr><td></td><td></td><td>KR850001491B</td><td>11.10.1985</td></tr>
<tr><td></td><td></td><td>JP63009359B</td><td>27.02.1988</td></tr>
<tr><td></td><td></td><td>JP1463794C</td><td>28.10.1988</td></tr>
<tr><td>US4819255A</td><td>04.04.1989</td><td>DE3738464A</td><td>01.06.1988</td></tr>
<tr><td></td><td></td><td>JP63127741A</td><td>31.05.1988</td></tr>
<tr><td>JP2005168601A</td><td>30.06.2005</td><td>None</td><td></td></tr>
<tr><td>CN201018708Y</td><td>06.02.2008</td><td>None</td><td></td></tr>
</table>

Form PCT/ISA/210 (patent family annex) (April 2007)

# INTERNATIONAL SEARCH REPORT

International application No.

PCT/CN2008/070664

A. CLASSIFICATION OF SUBJECT MATTER

A61B6/02(2006.01) i
G03B42/02(2006.01) i